# EUROPEAN PATENT APPLICATION

(11) **EP 4 470 476 A1**
(43) Date of publication of application: **04.12.2024**
(21) Application number: 23747217.0
(22) Date of filing: 13.01.2023
(51) Int. Cl.: A61B 10/00, A61B 10/04, A61B 1/04, A61B 1/273, A61B 1/31

(54) **MICROBIOME COLLECTION DEVICE**

(30) Priority: 28.01.2022 KR 20220013446
(71) Applicant: Soonchunhyang University Industry Academy Cooperation Foundation, Asan-si, Chungcheongnam-do 31538 (KR)
(72) Inventor: RYU, Chang Beom, Seongnam-si, Gyeonggi-do 13458 (KR); KIM, Young Ho, Bucheon-si, Gyeonggi-do 14547 (KR)
(74) Representative: Plougmann Vingtoft a/s
(86) International application number: PCT/KR2023/000637
(87) International publication number: WO 2023/146175

(57) **Abstract**

The present invention relates to a microbiome collection device comprising: a casing having an arrangement space and an inlet hole, and an absorption member, which is arranged in the arrangement space so as to absorb a microbiome that flows in through the inlet hole. Therefore, a microbiome is directly collected from the digestive organs without collecting the microbiome from feces excreted from the body, and thus analysis efficiency of a digestive organ microbiome is enhanced.

## Description

### Technical Field

This patent application claims priority to Korean Patent Application No. 10-2022-0013446, filed with the Korean Intellectual Property Office on January 28, 2022, the disclosure of which is incorporated herein by reference.

The present invention relates to a microbiome collection device.

### Background Art

Intestinal microorganisms have recently been actively studied due to confirmation of their association with diseases such as obesity and diabetes. In particular, they have recently been found to have a high correlation with brain diseases such as depression, autism, and Alzheimer's, and they are greatly variable according to acquired factors such as sleep, diet, alcohol, caffeine, and exercise, so that active research is underway to elucidate the mechanism of action in the human body in relation to the microbiome.

Feces are often used in microbiome research. Therefore, methods of collecting, purifying, and analyzing fecal samples are usually used to conduct research.

However, research results show that microbiome differs between various tissues in the intestines and feces, and the microbiome of feces has limitations in evaluating the composition and function of the intestinal microbiome.

### Disclosure of Invention

### Technical Problem

The present invention provides a technology to directly collect a microbiome while travelling along the digestive organs.

### Solution to Problem

A microbiome collection device according to an embodiment of the present invention includes a casing having an arrangement space and an inlet hole formed therein, and an absorption member arranged in the arrangement space so as to absorb a microbiome flowing in through the inlet hole.

The absorption member may be any one selected from the group consisting of a bio-sponge, a bio-organoid, bio, pulp, fabric, and a combination thereof.

The microbiome collection device may further include a membrane arranged in the inlet hole.

At least one partition wall arranged inside the casing so as to divide the arrangement space may be further included.

The absorption member may be independently arranged in each of the divided arrangement spaces, and a plurality of inlet holes may be formed in the casing so as to connect the respective divided arrangement spaces with the outside of the casing.

The microbiome collection device may further include a film that is selectively formed in the plurality of inlet holes and is meltable.

The pH of the film may be 6 to 6.5.

The microbiome collection device may further include a piston that is movably arranged inside the casing so as to pressurize the absorption member.

A rod hole into which a rod is inserted may be formed in the casing, and a circumferential diameter of the rod hole may be smaller than a circumferential diameter of the piston.

The microbiome collection device may further include a lens arranged in an open part of the casing, a camera module arranged inside the casing, and a controller arranged inside the casing and connected to the camera module.

### Advantageous Effects of Invention

According to an embodiment of the present invention, the microbiome collection device collects a microbiome directly from the digestive organs, instead of collecting a microbiome from feces excreted from the human body, thereby improving the efficiency of analyzing the microbiome of the digestive organs.

According to an embodiment of the present invention, since the membrane arranged in the inlet hole acts as a filter, undigested solid food does not flow into the inside of the casing, and only fluid flows into the same.

According to an embodiment of the present invention, films having a pH of 6 to 6.5 are selectively arranged in a plurality of inlet holes. The film is not decomposed in the stomach among the digestive organs, but is decomposed in the small intestine and large intestine. Therefore, digestive organ substances in the small intestine and large intestine can flow into the inside of the casing and be stored therein. On the other hand, digestive organ substances in the stomach can flow into the inlet hole where the film is not provided. Therefore, when the microbiome collection device passes through the digestive organs, digestive organ substances from the stomach, small intestine, and large intestine can be separately stored. Accordingly, microbiome analysis can be performed for each digestive organ.

According to an embodiment of the present invention, after obtaining the microbiome collection device discharged from the human body, when an analyst pressurizes the piston thereof, the piston can pressurize the absorption member while moving from one side to the other side in the casing. The digestive organ substances stored in the absorption member can be discharged to the outside of the casing through the inlet hole. Therefore, the workability of recovering digestive organ substances by the microbiome collection device is improved.

According to an embodiment of the present invention, since the microbiome collection device can collect and store substances of digestive organ while photographing digestive organs by the camera module and the controller arranged inside the casing, it is possible to check the digestive organs and collect a microbiome, thereby increasing the usability of medical personnel.

### Brief Description of Drawings

FIG. 1 is a schematic diagram illustrating a microbiome collection device according to an embodiment of the present invention.
FIG. 2 is a schematic diagram illustrating a microbiome collection device according to another embodiment of the present invention.
FIG. 3 is a schematic diagram illustrating a microbiome collection device according to another embodiment of the present invention.
FIG. 4 is a schematic diagram illustrating a microbiome collection device according to another embodiment of the present invention.
FIG. 5 is a schematic diagram illustrating a microbiome collection device according to another embodiment of the present invention.
FIG. 6 is a schematic diagram illustrating a microbiome collection device according to another embodiment of the present invention.
FIG. 7 is a schematic diagram illustrating a microbiome collection device according to another embodiment of the present invention.

### Best Mode for Carrying out the Invention

A microbiome collection device including a casing having an arrangement space and an inlet hole formed thereon, and an absorption member arranged in the arrangement space so as to absorb a microbiome that flows in through the inlet hole.

### Mode for Carrying out the Invention

Hereinafter, embodiments of the present invention will be described in detail with reference to the attached drawings so that those skilled in the art to which the present invention pertains are able to easily implement the invention. However, the present invention may be implemented in various different forms and is not limited to the embodiments described herein. Similar elements are denoted by the same reference numerals throughout the specification.

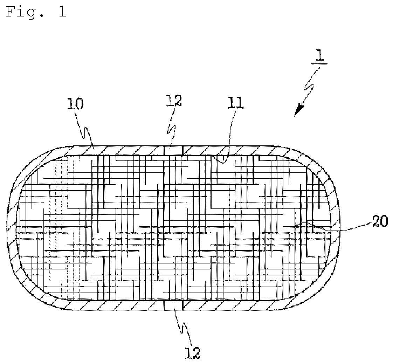

Then, a microbiome collection device according to an embodiment of the present invention will be described with reference to FIG. 1.

FIG. 1 is a schematic diagram illustrating a microbiome collection device according to an embodiment of the present invention.

Referring to FIG. 1, the microbiome collection device 1 according to the present embodiment includes a casing 10 and an absorption member (microbiome absorb biomaterials) 20, and moves along the digestive organs through oral administration to directly collect substances of digestive organs. Here, the substances of digestive organs may be things remaining in the digestive organ, such as fluids, solids, microbiomes, and the like.

The casing 10 forms the outer shape of the microbiome collection device 1 and is formed in a cylindrical shape with both ends in the longitudinal direction formed in a hemispherical shape. However, the outer shape of the casing 10 may be formed in a cylindrical shape and both ends in the longitudinal direction may be formed in a flat surface, and the edges connecting the outer circumference and the side may be formed in curved surfaces. Accordingly, when the microbiome collection device 1 passes through the digestive organs, the outer surface of the casing 10 does not cause damage to the digestive organs.

An arrangement space 11 is formed inside the casing 10. The casing 10 has at least one inlet hole 12 formed thereon to connect the arrangement space 11 to the outside. When the microbiome collection device 1 moves through the digestive organs, the substances of digestive organs may be introduced into the arrangement space 11 through the inlet hole 12.

The casing 10 may be made of a material that does not decompose while moving through the digestive organs. The casing 10 may be made of natural resin or synthetic resin, etc.

Meanwhile, the casing 10 may have a coating layer (not shown) formed on the outer surface thereof, which is made of a fluorescent material. Therefore, the microbiome collection device 1 discharged from the human body along with the feces may be easily found because it is distinguished from the feces in colors.

The absorption member (microbiome absorb biomaterials) 20 is arranged in the arrangement space 11 and protected. The absorption member 20 may absorb and store the substances of digestive organs that flow into the arrangement space 11. The absorption member 20 may have pores formed thereon.

The absorption member 20 may be a bio-sponge. However, the absorption member 20 may be a bio-organoid, bio pulp, fabric, etc. Various materials may be applied to the absorption member 20 as long as they are able to absorb the substances of digestive organs.

Meanwhile, although FIG. 1 shows that the inside of the casing 10 is filled with the absorption member 20, there may be a predetermined space between the absorption member 20 and the inner circumference of the casing 10.

If the microbiome collection device 1 according to the present embodiment is ingested orally, it moves along the digestive organs. At this time, the substances of digestive organs may flow into the inside of the casing 10 through the inlet hole 12. The absorption member 20 may absorb and store the substances of digestive organs. The microbiome collection device 1 may be excreted through the anus. Accordingly, microbiome is collected directly from the digestive organs, instead of being collected from feces, thereby improving the efficiency of analyzing the microbiome of the digestive organs.

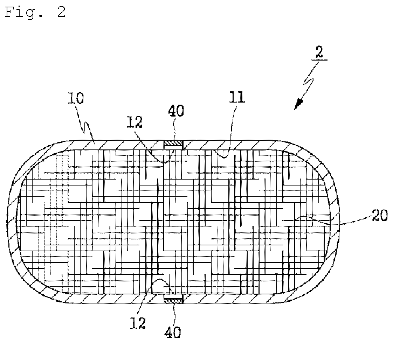

Next, a microbiome collection device according to another embodiment of the present invention will be described with reference to FIG. 2.

FIG. 2 is a schematic diagram illustrating a microbiome collection device according to another embodiment of the present invention.

Referring to FIG. 2, the microbiome collection device 2 according to the present embodiment includes a casing 10, an absorption member 20, and a membrane 40.

The casing 10 and the absorption member 20 according to the present embodiment have the same configuration and function as the casing and the absorption member according to the embodiment in FIG. 1, so redundant descriptions thereof will be omitted.

The membrane 40 is formed in the inlet hole 12. The membrane 40 may be made of a breathable non-woven fabric. The membrane 40 has permeability so that fluids may pass therethrough while solids are blocked. The membrane 40 acts as a filter so that undigested solid food does not flow into the inside of the casing 10, and only fluids flow thereinto.

Other configurations may be applied as is to the configuration of the embodiment in FIG. 1.

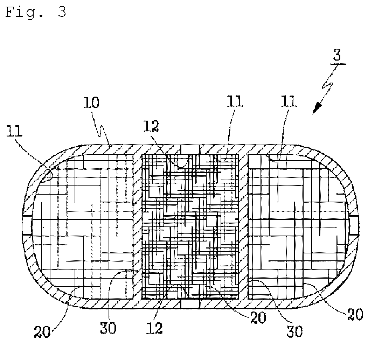

Next, a microbiome collection device according to another embodiment of the present invention will be described with reference to FIG. 3.

FIG. 3 is a schematic diagram illustrating a microbiome collection device according to another embodiment of the present invention.

Referring to FIG. 3, the microbiome collection device 3 according to the present embodiment includes a casing 10, an absorption member 20, and a partition wall 30.

The casing 10 and the absorption member 20 according to the present embodiment have the same configuration and function as the casing and the absorption member according to the embodiment in FIG. 1, so redundant descriptions thereof will be omitted.

The partition wall 30 is arranged inside the casing 10 so as to be formed integrally with the casing 10. The arrangement space 11 is divided into a plurality of spaces by the partition walls 30. Although FIG. 3 shows two partition walls 30 and three arrangement spaces 11, the number of partition walls 30 and the number of arrangement spaces 11 may vary depending on the design of the microbiome collection device 3.

The absorption member 20 is independently arranged in each arrangement space 11, and a plurality of inlet holes 12 are formed in the casing 10 to connect the arrangement spaces 11 to the outside of the casing 10. Accordingly, the substances of digestive organs may flow into the respective arrangement spaces 11.

Other configurations may be applied as is to the configuration of the embodiment in FIG. 1.

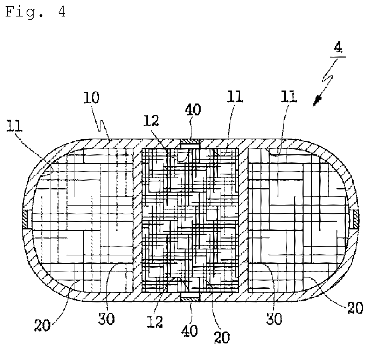

Next, a microbiome collection device according to another embodiment of the present invention will be described with reference to FIG. 4.

FIG. 4 is a schematic diagram illustrating a microbiome collection device according to another embodiment of the present invention.

Referring to FIG. 4, the microbiome collection device 4 according to the present embodiment includes a casing 10, an absorption member 20, a partition wall 30, and a membrane 40.

The casing 10, the absorption member 20, and the partition wall 30 according to the present embodiment have the same configuration and function as the casing, the absorption member, and the partition wall according to the embodiment in FIG. 3, so redundant descriptions thereof will be omitted.

The membrane 40 is formed in the inlet hole 12. The membrane 40 may be made of a breathable non-woven fabric. The membrane 40 has permeability so that fluids may pass therethrough while solids are blocked. The membrane 40 acts as a filter so that undigested solid food does not flow into the inside of the casing 10, and only fluids flow thereinto.

Other configurations may be applied as is to the configuration of the embodiment in FIG. 3.

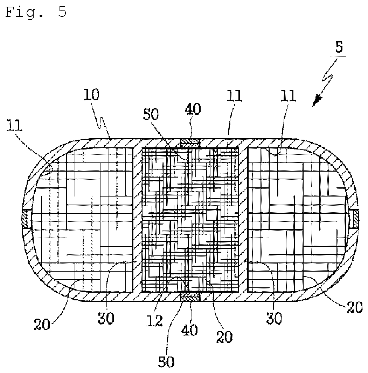

Next, a microbiome collection device according to another embodiment of the present invention will be described with reference to FIG. 5.

FIG. 5 is a schematic diagram illustrating a microbiome collection device according to another embodiment of the present invention.

Referring to FIG. 5, the microbiome collection device 5 according to the present embodiment includes a casing 10, an absorption member 20, a partition wall 30, a membrane 40, and a film 50.

The casing 10, the absorption member 20, the partition wall 30, and the membrane 40 according to the present embodiment have the same configuration and function as the casing, the absorption member, the partition wall, and the membrane according to the embodiment in FIG. 4, so redundant descriptions thereof will be omitted.

The film 50 is formed in one selected from among a plurality of inlet holes 12 formed in the casing 10. The film 50 may block the substances of digestive organs flowing into the inlet hole 12 from entering the arrangement space 11. However, the film 50 may be decomposed by the substances of digestive organs. The pH of the film may be 6 to 6.5. Therefore, when the microbiome collection device 5 passes through the stomach, the film 50 may not be decomposed, but may be decomposed while passing through the small intestine and large intestine.

When the microbiome collection device 5 passes through the stomach, the substances of digestive organs may pass through the membrane 40 and flow into the arrangement space 11. Accordingly, the absorption member 20 may absorb and store the substances of digestive organs in the stomach.

On the other hand, when the microbiome collection device 5 passes through the small intestine and large intestine, the film 50 may be decomposed, so that the substances of digestive organs in the small intestine and large intestine may pass through the membrane 40 and be absorbed and stored in the absorption member 20.

Therefore, when the microbiome collection device 5 passes through the digestive organs, the substances of digestive organs such as the stomach, small intestine, and large intestine may be separately stored. Accordingly, it is possible to analyze a microbiome for each digestive organ.

Meanwhile, the membrane 40 and film 50 of the inlet hole 12 may be omitted, and a coating liquid having a pH of 6 to 6.5 may be formed in one selected absorption member to form a coating layer.

The coating layer may not be decomposed when the microbiome collection device 5 passes through the stomach, but may be decomposed when the microbiome collection device 5 passes through the small intestine and large intestine. Accordingly, the substances of digestive organs in the small intestine and large intestine may be absorbed and stored in the absorption member on which the coating layer is formed.

Other configurations may be applied as is to the configuration of the embodiment in FIG. 4.

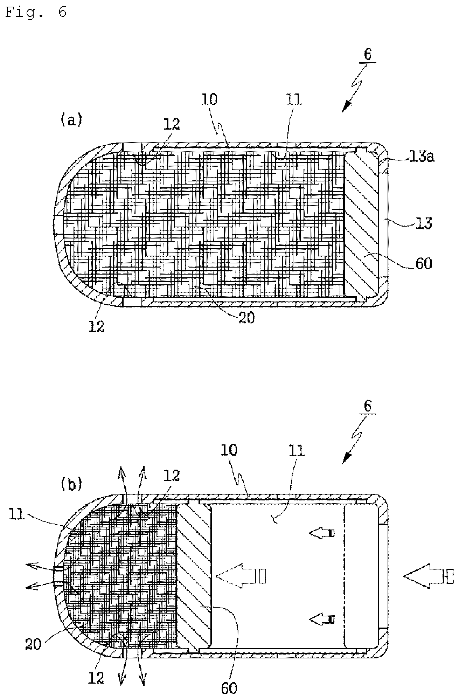

Next, a microbiome collection device according to another embodiment of the present invention will be described with reference to FIG. 6.

Fig. 6 is a schematic diagram illustrating a microbiome collection device according to another embodiment of the present invention, in which a) shows a state in which a piston does not pressurize the absorption member, and b) shows a state in which the piston pressurizes the absorption member.

Referring to FIG. 6, the microbiome collection device 6 according to the present embodiment includes a casing 10, an absorption member 20, and a piston 60.

The casing 10 and the absorption member 20 according to the present embodiment have the same configuration and function as the casing and the absorption member according to the embodiment in FIG. 1, so redundant descriptions thereof will be omitted. However, the casing 10 according to the present embodiment has an opening 13 formed on one side. The diameter of the opening 13 is smaller than the inner circumferential diameter of the casing 10. Accordingly, a stop jaw 13a is formed on one inner side of the casing 10.

The piston 60 is arranged on one inner side of the casing 10. The circumference of the piston 60 is in contact with the inner circumference of the casing 10. The piston 60 may move from one side of the casing 10 to the other side by the pressure of the load flowing in through the opening 13. The absorption member 20 may be compressed by the piston 60.

Meanwhile, a guide groove and a guide rail are formed between the outer circumference of the piston 60 and the inner circumference of the casing 10. A plurality of guide grooves and a plurality of guide rails are formed at intervals along the circumferential direction. The guide grooves and guide rails guide the piston 60 to move in a straight line without rotating.

As shown in a) of FIG. 6, the substances of digestive organs are absorbed and stored in the absorption member 20 of the microbiome collection device 6 discharged from the human body, and the piston 60 is located on one inner side.

After obtaining, when the analyst pressurizes the piston 60, the piston 60 may pressurize the absorption member 20 while moving from one inner side of the casing 10 to the other side as shown in b) of FIG. 6. The substances of digestive organs stored in the absorption member 20 may be discharged to the outside of the casing 10 through the inlet hole 12. Accordingly, the workability of recovering the substances of digestive organs from the microbiome collection device 6 is improved.

Other configurations may be applied as is to the configuration of the embodiment in FIG. 1.

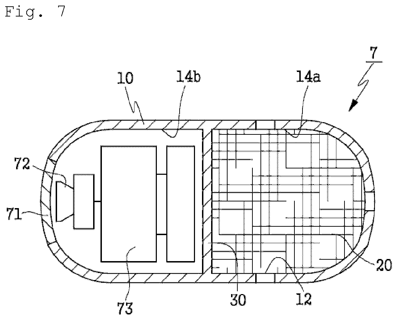

Next, a microbiome collection device according to another embodiment of the present invention will be described with reference to FIG. 7.

FIG. 7 is a schematic diagram illustrating a microbiome collection device according to another embodiment of the present invention.

Referring to FIG. 7, the microbiome collection device 7 according to the present embodiment includes a casing 10, an absorption member 20, a partition wall 30, a lens 71, a camera module 72, and a controller 73.

The casing 10, the absorption member 20, and the partition wall 30 according to the present embodiment have the same configuration and function as the casing 10, the absorption member 20, and the partition wall 30 according to the embodiments in FIGS. 3 to 5, so redundant descriptions thereof will be omitted. Here, the interior of the casing 10 is divided into an absorption space 14a and a module space 14b by the partition wall 30, and the absorption member 20 is arranged in the absorption space 14a, and the camera module 72 and the controller 73 are arranged in the module space 14b. At least one inlet hole 12 is formed in the part of the casing 10 corresponding to the absorption space 14a.

The lens 71 is formed in the part of the casing 10 corresponding to the module space 14b. The lens 71 may be configured by forming a portion of the casing 10 to be transparent. However, a portion of the casing 10 may be open, and the lens 71 may be detachably coupled to the open portion. The lens 71 may be a flat lens.

The camera module 72 and the controller 73 are connected to each other, and the controller 73 may include a power supply, a communication unit, and a circuit unit. The camera module 72 may capture the outside of the casing 10 through the lens 71. The controller 73 may store the captured image of the camera module 72 or transmit the same to a management server through the communication unit. The controller 73 may also store the captured images every minute.

Therefore, since the microbiome collection device 7 is able to collect and store substances of digestive organs while photographing digestive organs, it is possible to check the digestive organs and collect a microbiome, thereby increasing the usability of medical personnel.

Meanwhile, the microbiome collection device according to the present invention may be mounted to the tip of a known endoscope and inserted into the human body through the mouth or anus, thereby absorbing and storing the substances of digestive organs.

Other configurations may be applied as is to the configurations of the embodiments in FIGS. 3 to 5.

Although the preferred embodiments of the present invention have been described in detail above, the scope of the present invention is not limited thereto, and various modifications and improvements made by those skilled in the art using the basic concept of the present invention defined in the claims below also fall within the scope of the present invention.

**[Description of Reference numerals]**

| | | | |
|---|---|---|---|
| 1, 2, 3, 4, 5, 6, 7: | Microbiome collection device | | |
| 10: | Casing | 11: | Arrangement space |
| 12: | Inlet hole | 13: | Opening |
| 13a: | Stop jaw | 14a: | Absorption space |
| 14b: | Module space | 20: | Absorption member |
| 30: | Partition wall | 40: | Membrane |
| 50: | Film | 60: | Piston |
| 71: | Lens | 72: | Camera module |
| 73: | Controller | | |

### Industrial Applicability

The present invention relates to a microbiome collection device.

## Claims

1. A microbiome collection device comprising
a casing having an arrangement space and an inlet hole formed therein, and
an absorption member arranged in the arrangement space so as to absorb a microbiome flowing in through the inlet hole.

2. The microbiome collection device according to claim 1,
wherein the absorption member is any one selected from the group consisting of a bio-sponge, a bio-organoid, bio, pulp, fabric, and a combination thereof.

3. The microbiome collection device according to claim 1,
further comprising a membrane arranged in the inlet hole.

4. The microbiome collection device according to claim 1 or claim 3,
further comprising at least one partition wall arranged inside the casing so as to divide the arrangement space,
wherein the absorption member is independently arranged in each of the divided arrangement spaces, and
wherein a plurality of inlet holes are formed in the casing so as to connect the respective divided arrangement spaces with the outside of the casing.

5. The microbiome collection device according to claim 4,
further comprising a film that is selectively formed in the plurality of inlet holes and is meltable.

6. The microbiome collection device according to claim 5,
wherein the pH of the film is 6 to 6.5.

7. The microbiome collection device according to claim 1,
further comprising a piston that is movably arranged inside the casing so as to pressurize the absorption member,
wherein a rod hole into which a rod is inserted is formed in the casing, and
wherein a circumferential diameter of the rod hole is smaller than a circumferential diameter of the piston.

8. The microbiome collection device according to claim 1, further comprising
a lens arranged in an open part of the casing,
a camera module arranged inside the casing, and
a controller arranged inside the casing and connected to the camera module.
